# EUROPEAN PATENT APPLICATION

(11) **EP 1 502 917 A1**
(43) Date of publication of application: **02.02.2005**
(21) Application number: 03017056.7
(22) Date of filing: 28.07.2003
(51) Int. Cl.: C07D 471/04, C07D 213/90, G11B 7/24

(54) **Use of mono- and dicationic naphthalene-based imide dyes in optical layers for optical data recording**

(71) Applicant: CLARIANT INTERNATIONAL LTD., 4132 Muttenz (CH)
(72) Inventor: Graciet, Jean-Christophe, 68330 Huningue (FR)

(57) **Abstract**

The present invention relates to the use of mono- and dicationic naphthalene-based imide dyes known as charge control agents, antistatic agents or optical brighteners as dyes in optical layers for optical data recording.

In particular, the present invention relates to optical layers comprising as dyes mono- or di-cationic naphthalene tetracarboxylic di-imide with high-performance anions as counterions, such as tetracyanoquinodimethane (TCNQ-), tetraphenylborate or hexafluoroantimonate.

The invention further relates to a write only read many (WORM) type optical recording media capable of recording and reproducing information with radiation of blue laser, which comprises a mono- or di-cationic naphthalene tetracarboxylic di-imide type dye in the optical layer.

## Description

The present invention relates to the use of mono- and dicationic naphthalene-based imide dyes known as charge control agents, antistatic agents or optical brighteners as dyes in optical layers for optical data recording.

Recently, organic dyes have attracted considerable attentions in the field of diode-laser optical storage. Commercial recordable compact discs (CD-R) and recordable digital versatile discs (DVD-R) can contain, as recording layer, numerous dyes based on phthalocyanine, hemicyanine, cyanine and metallized azo structures. These dyes are suitable in their respective fields with the laser wavelength criteria. Other general requirements for dye media are strong absorption, high reflectance, high recording sensitivity, low thermal conductivity as well as light and thermal stabilities, durability for storage and non-toxicity.

For industrial application, organic dyes in the field of diode-laser optical storage have to be suitable for the spin coating process to prepare thin films, i.e. they have to be sufficiently soluble in the organic solvents generally applied in the spin coating process.

Write only read many (WORM) type and erasable type optical recording media reproduce information by detecting variations in the reflectivity caused by physical deformation, by alterations of optical characteristics as well as by phase properties and magnetic properties of a recording layer before and after the recording.

Recordable compact discs with a storage capabilities up to 680 MBytes (CD-R) are an example of a WORM type optical recording medium. Recently, digital versatile discs (DVD) with increased information storage capabilities up to 4.7 GBytes have been commercialized and DVD-R are the next logical step.

The DVD-R technology adopts as a light source a red diode laser with a wavelength of 630-670 nm. Thereby the pit size and track interval can be reduced, increasing the information storage capacity by up to 6-8 times compared to CD-R's.

Blu-ray® discs (Blu-ray® disc is a standard developed by Hitachi Ltd., LG Electronics Inc., Matsushita Electric Industrial Co. Ltd., Pioneer Corporation, Royal Philips Electronics, Samsung Electronics Co. Ltd., Sharp Corporation, Sony Corporation, Thomson Multimedia) are going to be the next milestone in optical recording technology. Its new specification increases the data storage up to 27 GBytes per recording layer for a 12 cm diameter disc. By adopting a blue diode laser with a wavelength of 405 nm (e.g. by use of GaN or SHG laser diodes), the pit size and track interval can be further reduced, again increasing the storage capacity by an order of magnitude.

The construction of optical data recording media is known in the art. An optical data recording media generally comprises a substrate and a recording layer, the optical layer. Usually discs or wavers of organic polymeric materials are used as substrates. Preferred substrates are polycarbonate (PC) or polymethylmethacrylate (PMMA). The substrate has to provide an even and uniform surface of high optical quality. The optical layer is deposited thereon in a thin and uniform film of high optical quality and defined thickness. Finally, a reflective layer, e.g. aluminium, gold or copper, is deposited upon the optical layer.

Advanced optical data recording media may comprise further layers, such as protective layers, adhesive layers or additional optical layers.

To provide for a thin and uniform film of the optical layer, the material is usually deposited by spin coating, vacuum evaporation, jet coating, rolling coating, or soaking. The preferred process in industry is spin coating to form an optical layer of about 70 nm to 250 nm thickness. For the application in the spin coating process, the material of the optical layer has to be highly soluble in organic solvents.

Monocationic naphthalimide, mono- or dicationic naphthalene tetracarboxylic di-imide type dyes are known in the art for various applications.

**DE4040468** (Hoechst AG), **DE4040469** (Hoechst AG), **DE3604827** (Bayer AG) and **DE 3738948** (Bayer AG) disclose quaternised naphthalene dicarboxylic imide (naphthalimide) and naphthalene tetracarboxylic di-imide and their use as charge control agents in toners.

**EP224779** (Bayer AG) disclose the same general structures for the use as antistatic agents.

**DE3535496** (Bayer AG) discloses naphthalene tetracarboxylic di-imide compounds as fluorescence quenchers and **DE1419350** (Kasai) discloses their use as fluorescent brightening agent for fiber application.

The general structures known in the art and disclosed in the above mentioned patents are the following: 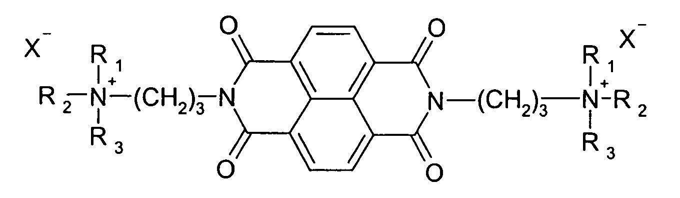 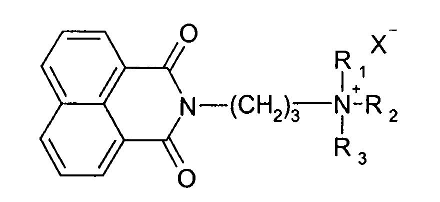 wherein X⁻ are conventional anions.

Further derivatives of quaternised naphthalene dicarboxylic imide (naphthalimide) and naphthalene tetracarboxylic di-imide known in the art are the following:
**Heywang et al.** discloses the following compound useful due to its electric conductivity (Angewandte Chemie **1989**, 101, 462-4).

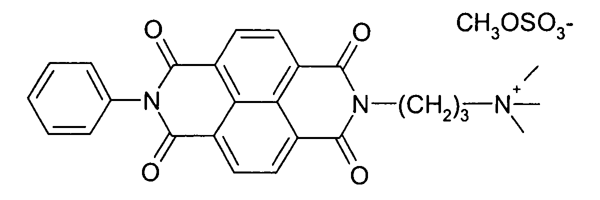

**JP39027124** (Sumitomo) discloses the following compound useful as cationic dyes for polyacrylonitrile fibres:

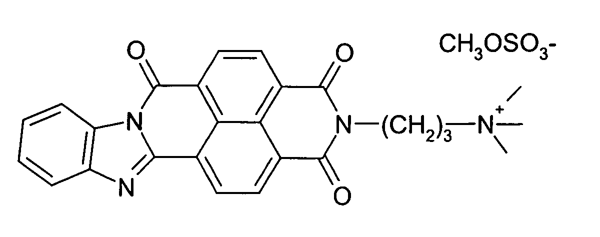

Surprisingly it has now been found that mono-cationic naphthalimide and mono- or di-cationic naphthalene tetracarboxylic di-imide type dyes are useful as dye compounds in optical layers for optical recording media.

The present invention therefore in general terms relates to optical layers comprising mono-cationic naphthalimide and mono- or di-cationic naphthalene tetracarboxylic di-imide type dyes as dyes and the use of said optical layers for optical recording media.

More particularly, the invention relates to mono-cationic naphthalimide and mono- or di-cationic naphthalene tetracarboxylic di-imide type dyes, to an optical layer comprising said dyes and to a write only read many (WORM) type optical recording medium capable of recording and reproducing information with radiation of blue laser of preferably 405 nm, which employs a mono-cationic naphthalimide or a mono- or di-cationic naphthalene tetracarboxylic di-imide dye type in the optical layer.

Surprisingly it has been found, that the performance of cationic dyes for optical layers derived from compounds known as charge control agents, antistatic agents or optical brighteners with conventional anions as counterions, such as halide (fluoride, chloride, bromide, iodide), C₁₋₂₀ carboxylate, formiate, acetate, oxalate, lactate, glycolate, citrate or methylsulfate, can be significantly improved by exchanging the conventional anions for high-performance anions, such as tetracyanoquinodimethane (TCNQ⁻), BX₄⁻, SbX₆⁻, PX₆⁻ in which X represents halogen (-F, -Cl, -Br, -I) or phenyl.

Therefore in a preferred embodiment, the invention relates to mono-cationic naphthalimide and mono- or di-cationic naphthalene tetracarboxylic di-imide type dye with high-performance anions as counterions selected from the group consisting of tetracyanoquinodimethane (TCNQ⁻), BX₄⁻, SbX₆⁻ and PX₆⁻ in which X represents halogen (-F, -Cl, -Br, -I) or phenyl.

In a more preferred embodiment, the invention relates to mono-cationic naphthalimide and mono- or di-cationic naphthalene tetracarboxylic di-imide type dye with high-performance anions as counterions selected from the group consisting of tetracyanoquinodimethane (TCNQ⁻), tetraphenylborate (BPh₄⁻) and hexafluoroantimonate (SbF₆⁻).

The present invention is directed to the use of a mono- or dicationic naphthalene-based imide dye compound of the formulae (I), (II), (III) or (IV) in an optical layer for optical data recording, wherein the dye compound is selected from the group consisting of compounds of the formulae (I), (II), (III) and (IV). 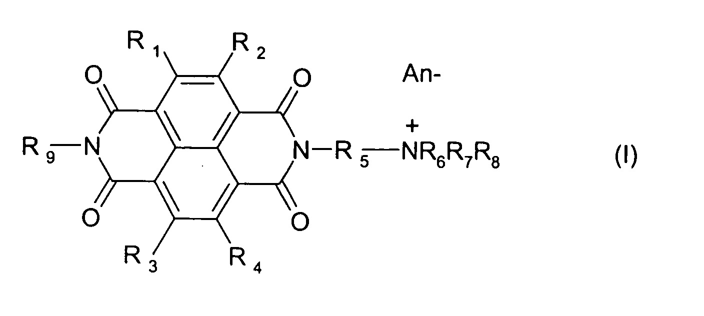 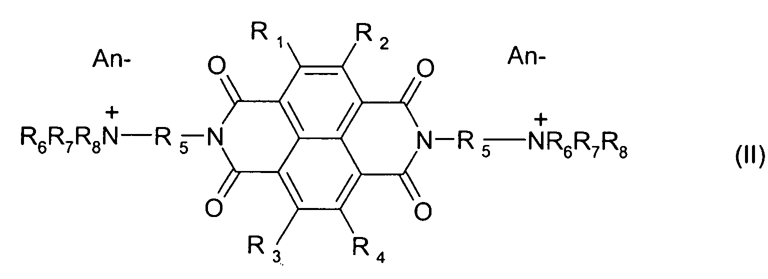 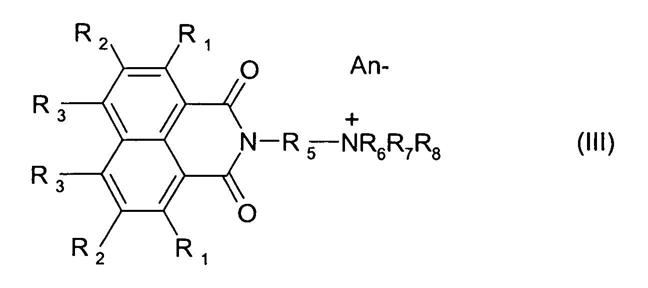 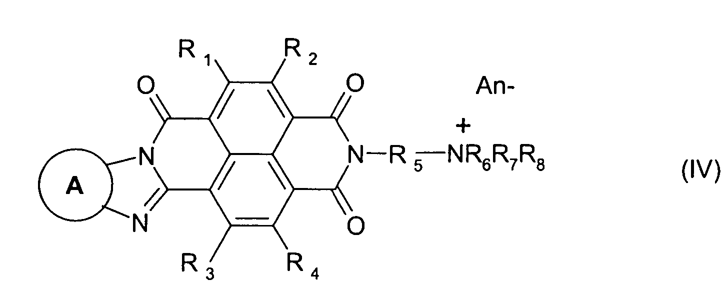 wherein
R₁, R₂, R₃, R₄ independently of one another, represent hydrogen, hydroxy (-OH), halogen (-F, -Cl, -Br, -I), cyano (-CN), nitro (-NO₂), C₁₋₈ alkyl, C₆₋₁₂ aryl, -NR₁₁R₁₂, C₁₋₈ alkyl, C₆₋₁₂ aryl, C₁₋₈ alkoxy, C₁₋₈ alkylthio, C₆₋₁₂ aryloxy, C₆₋₁₂ arylthio, -SO₃H, -SO₂R₁₁, -SO₂NR₁₁R₁₂, -CO₂R₁₁, - CONR₁₁R₁₂, -NHCOR₁₁, in which R₁₁ and R₁₂ are independently hydrogen or C₁₋₈ alkyl;
R₅ is C₁₋₂₀ alkylene, wherein the alkylene can be unsubstituted or substituted by C₁₋₈ alkoxy, C₁₋₈ alkylthio, C₆₋₁₂ aryloxy, C₆₋₁₂ arylthio, C₆₋₁₂ aryl;
R₆ is C₁₋₂₀ alkyl, wherein the alkyl can be unsubstituted or substituted by C₁₋₈ alkoxy, C₁₋₈ alkylthio, C₆₋₁₂ aryloxy, C₆₋₁₂ arylthio, C₆₋₁₂ aryl;
R₇, R₈ independently of one another, represent C₁₋₂₀ alkyl, wherein the alkyl can be unsubstituted or substituted by C₁₋₈ alkyl, C₁₋₈ alkoxy, C₁₋₈ alkylthio, C₆₋₁₂ aryloxy, C₆₋₁₂ arylthio, C₆₋₁₂ aryl; or are combined together to form C₃₋₆ cycloalkyl which can be unsubstituted or substituted by C₁₋₈ alkoxy, C₁₋₈ alkylthio, C₆₋₁₂ aryloxy, C₆₋₁₂ arylthio, C₆₋₁₂ aryl;
R₉ is C₁₋₂₀ alkyl wherein the alkyl can be unsubstituted or substituted by C₁₋₈ alkyl, C₁₋₈ alkoxy, C₁₋₈ alkylthio, C₆₋₁₂ aryloxy, C₆₋₁₂ arylthio, C₆₋₁₂ aryl; or R₉ is C₆-aryl wherein the aryl can be unsubstituted or substituted by C₁₋₈ alkyl, C₁₋₈ alkoxy, C₁₋₈ alkylthio, C₆₋₁₂ aryloxy, C₆₋₁₂ arylthio; or R₉ represents a substituted or unsubstituted annealed aromatic ring system comprising at least one heteroatom in either the first or the second ring or in both, selected from one of the moieties (1) to (4):

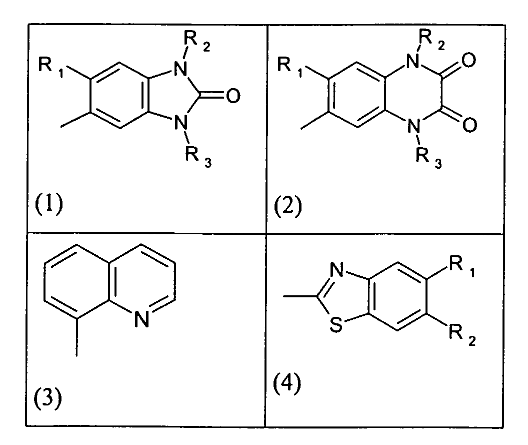

Nucleus A represents a substituted or unsubstituted annealed aromatic ring system comprising at least one heteroatom in either the first or the second ring or in both. A is selected from one of the moieties (5) to (8):

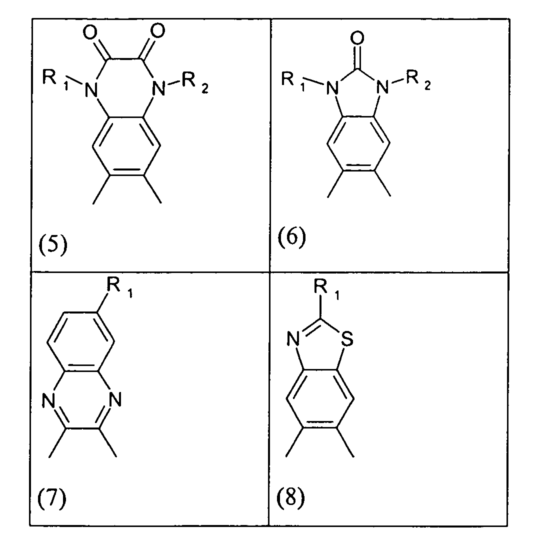

An⁻ represents an anion selected from halide, C₁₋₂₀ carboxylate, formiate, acetate, oxalate, lactate, glycolate, citrate, H₃C-SO₄⁻, ClO₄⁻, tetracyanoquinodimethane (TCNQ⁻), H₃C-SO₃⁻, ClO₄⁻, BX₄⁻, SbX₆⁻, PX₆⁻ in which X represents halogen (-F, -Cl, -Br, -I) or phenyl.

In a preferred aspect, the present invention relates to an optical layer comprising at leas one dye compound of the formulae (I), (II) , (III) or (IV) as described above, wherein
- R₁, R₂, R₃, R₄: independently of one another, represent hydrogen, halogen (-F, -Cl, - Br, -I), cyano (-CN), nitro (-NO₂), C₁₋₈ alkyl, C₆₋₁₂ aryl, hydroxy, C₁₋₈ alkoxy, C₆₋₁₂ aryloxy,
- R₅: is C₁₋₆ alkylene, wherein the alkylene can be unsubstituted or substituted by C₁₋₆ alkoxy;
- R₆: is C₁₋₆ alkyl, wherein the alkyl can be unsubstituted or substituted by C₁₋₈ alkoxy;
- R₇, R₈: independently of one another, represent C₁₋₈ alkyl, wherein the alkyl can be unsubstituted or substituted by C₁₋₈ alkyl, C₁₋₈ alkoxy; or are combined together to form C₃₋₆ cycloalkyl which can be unsubstituted or substituted by C₁₋₈ alkyl or C₁₋₈ alkoxy;
- R₉ is: is C₁₋₂₀ alkyl, wherein the alkyl can be unsubstituted or substituted by C₁₋₈ alkyl, C₁₋₈ alkoxy; or R₉ is phenyl being unsubstituted or substituted by C₁₋₈ alkyl, C₁₋₈ alkoxy; or R₉ is selected from one of the moieties (1) to (4) wherein R₁, R₂ and R₃ are defined as above;
- Nucleus A is: selected from one of the moieties (5) to (8), wherein R₁, R₂ and R₃ are defined as above;
- An': represents an anion selected from tetracyanoquinodimethane (TCNQ⁻), BX₄⁻, SbX₆⁻, PX₆⁻, in which X represents halogen (-F, -Cl, - Br, -I) or phenyl.

In one more preferred embodiment, the present invention relates to an optical layer comprising at least one dye compound of the formulae (I), (II), (III) or (IV) as described above, wherein
- R₁, R₂, R₃, R₄: three thereof represent hydrogen and one thereof represents C₁₋₄ alkoxy;
- R₅: is methylene, ethylene or propylene;
- R₆: is C₁₋₆ alkyl;
- R₇, R₈: represent C₁₋₈ alkyl or combined together to form with nitrogen a cyclobutylamine, cyclopentylamine, cyclohexylamine;
- R₉: is C₆₋₁₈ alkyl or methoxyphenyl or moieties (1) or (2) with R₁ being hydrogen and R₂ and R₃ being ethyl;
- Nucleus A: is selected from one of the moieties (5) and (6) with R₁ being hydrogen and R₂ and R₃ being ethyl;
- An⁻: represents an anion selected from tetracyanoquinodimethane (TCNQ⁻), BX₄⁻, SbX₆⁻, PX₆⁻, in which X represents fluoro or phenyl.

In the most preferred embodiment, the present invention relates to an optical layer comprising at least one dye compound of the formulae (I), (II), (III) or (IV) as described above, wherein
- R₁, R₂, R₃, R₄: three thereof represent hydrogen and one thereof represents C₁₋₄ alkoxy;
- R₅: is methylene, ethylene or propylene;
- R₆: is C₁₋₆ alkyl;
- R₇, R₈: represent C₁₋₈ alkyl or combined together to form with nitrogen a cyclobutylamine, cyclopentylamine, cyclohexylamine;
- R₉: is C₆₋₁₈ alkyl or methoxyphenyl or moieties (1) or (2) with R₁ being hydrogen and R₂ and R₃ being ethyl;
- Nucleus A: is selected from one of the moieties (5) and (6) with R₁ is hydrogen and R₂ and R₃ are ethyl;
- An⁻: represents an anion selected from tetracyanoquinodimethane (TCNQ⁻), SbF₆⁻.

An optical layer according to the invention may also comprise a mixture of two or more, preferably of two dye compounds according to formulae (I), (II), (III) or (IV) as defined above.

Further, the invention relates to a method for producing optical layers comprising the following steps
(a) providing a substrate
(b) dissolving a dye compound or a mixture of dye compounds of formulae (I), (II), (III) or (IV) in an organic solvent to form a solution,
(c) coating the solution (b) on the substrate (a);
(d) evaporating the solvent to form a dye layer.

Preferred substrates are polycarbonate (PC) or polymethylmethacrylate (PMMA).

Organic solvents are selected from C₁₋₈ alcohol or halogen substituted C₁₋₈ alcohols, C₁₋₈ ketone, C₁₋₈ ether, halogen substituted C₁₋₄ alkane, or amides.

Preferred C₁₋₈ alcohols or halogen substituted C₁₋₈ alcohols are for example methanol, ethanol, isopropanol, diacetone alcohol (DAA), 2,2,3,3-tetrafluoropropanol, trichloroethanol, 2-chloroethanol, octafluoropentanol or hexafluorobutanol.

Preferred C₁₋₈ ketones are for example acetone, methylisobutylketone, methylethylketone, or 3-hydroxy-3-methyl-2-butanone.

Preferred halogen substituted C₁₋₄ alkanes are for example chloroform, dichloromethane or 1-chlorobutane.

Preferred amides are for example dimethylformamide or dimethylacetamide.

The optical layer (dye layer) preferably has a thickness from 70 to 250 nm.

In a further aspect, the present invention provides for an optical layer suitable for a high-density recording medium, e.g. of the WORM disc format, in a laser wavelength range of from 350-450nm, preferably around 405 nm.

The dye compounds of formulae (I), (II), (III) or (IV) possess the required optical characteristics (such as high absortivity and high recording sensitivity), an excellent solubility in organic solvents, an excellent light stability and a decomposition temperature of 250-300°C.

The dye compounds of formulae (I), (II), (III) or (IV) are easy to synthesize with high yields and can be manufactured at a reduced cost. The general synthesis pathway is known in the art.

The patent applications cited above disclose a general process to obtain the desired mono-cationic naphthalimide and mono- or dicationic naphthalene tetracarboxylic di-imide dye type compounds, respectively of formulae (I), (II), (III) and (IV).

### Preparation of high density optical disc recording medium

The preparation of a high density optical recording medium / high density optical disc conventionally comprises the following steps:
(a) providing a first substrate
(b) dissolving the dye in an organic solvent to form a solution,
(c) coating the first solution on the first substrate;
(d) drying the solution to form a dye layer and
(e) disposing a reflection layer on the dye layer and
(f) disposing a second substrate on the reflection layer

In step (f), preferably a second substrate is bonded with the first substrate to form the high-density optical disc recording medium. Conventional techniques for bonding are printing, glueing or melting.

### EXAMPLES

All dyes are prepared using standard procedures known in the art. If required, the dyes can be purified by recrystallization from an alcohol.

The complex dyes are prepared by reaction of a solution of counter-ion salt (e.g. sodium hexafluoroantimonate, LiTCNQ, sodium tetraphenylborate) with a boiling or hot solution of the corresponding starting material in a selected alcohol. The precipitate is isolated following standard methods.

The synthesis of LiTCNQ is done according to L. R. Melby et al., J. Am. Chem. Soc., 84, p.3374, (1962).

### EXAMPLE 1

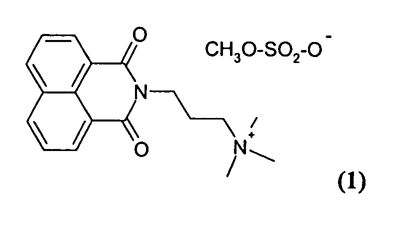

### EXAMPLE 2

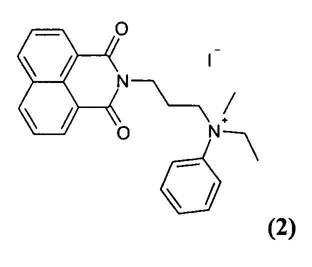

### EXAMPLE 3

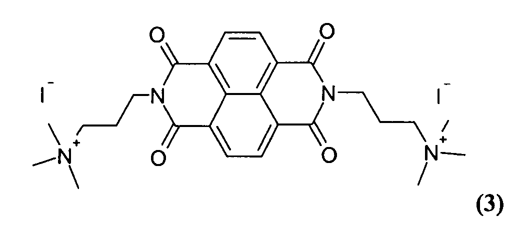

### EXAMPLE 4

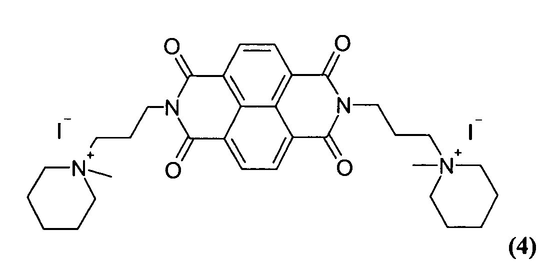

### EXAMPLE 5

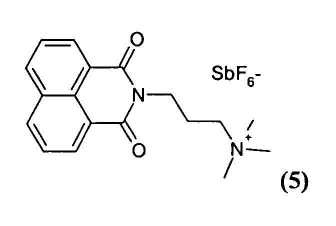

### EXAMPLE 6

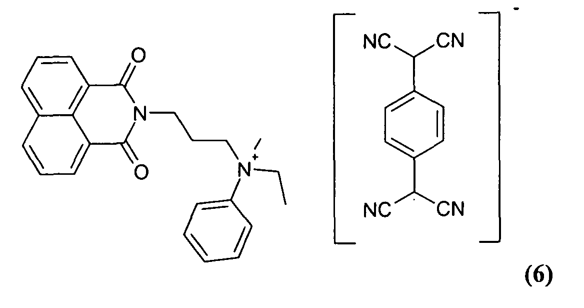

### EXAMPLE 7

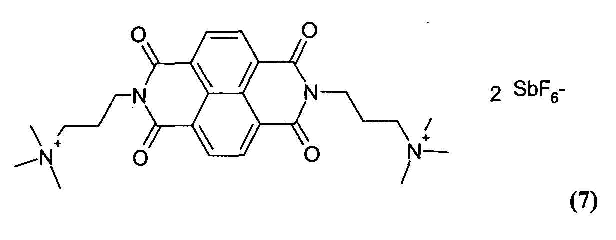

### OPTICAL CHARACTERISTICS (IN SOLUTION)

**TABLE 1**

| **Example** | **Anion** | **λ**_{**max**} | **ε (λ**_{**max**}**) I/mol.cm.** |
|---|---|---|---|
| **(1)** | CH₃OSO₂-O-⁻ | 335 nm | 12500 |
| **(2)** | I⁻ | 343 nm | 13500 |
| **(3)** | I⁻ | 382 nm | 10500 |
| **(4)** | I⁻ | 382 nm | 12000 |
| **(5)** | SbF₆⁻ | 340 nm | 17500 |
| **(6)** | TCNQ⁻ | 450 nm | 40000 |
| **(7)** | SbF₆⁻ | 350 nm | 18000 |

As the optical characteristics (in solution) of examples I to 7 clearly indicate, the exchange of conventional anions, such as iodide or methylsulfate (in examples 1, 2, 3 and 4) with high-performance anions, such as SbF₆⁻ or TCNQ⁻ (in examples 5, 6 and 7) significantly improves the ε (λₘₐₓ).

In particular the comparison of examples 3, 4 and 7 indicates, that structural alterations at the quarternized N-atom have a much smaller effect, than the exchange of the counter-ion form a conventional anion to a high-performance anion.

### EXAMPLE 8

0.30 g of the dye of formula (1) obtained from example 1 were dissolved in 10 ml of tetrafluoropropane, and stirred at room temperature for 5 hours. The solution was filtered with a Teflon filter (having 0,2 µm pore size) and spin coated over a substrate to form a dye layer. The substrate was a polycarbonate substrate with 0.6 mm thickness having pregrooves with 150 nm depth, 300 nm width and 800 nm track pitch.

The substrate having the dye layer was dried in a vacuum oven set at 40°C for 12 hours. The optical layers obtained had a uniform and even surface at a thickness of 120 nm.

As a reflective layer, silver was deposited over the dye layer with a thickness of 100 nm. An acrylic UV curable resin was spin-coated over the reflective layer and then cured by radiation of UV rays to form a protective layer.

Finally, the substrate with the reflective layer and the recording layer was combined with another blank substrate with a thickness of 0.6 mm. The final product is a high-density recordable optical disc of about 120 mm diameter.

To evaluate the final product a PULSTEC DDU-1000 evaluation test machine was used to write and read the test results.

The recording conditions were: constant linear velocity (CLV) at 3.5 m/s, wavelength at 405 nm, numerical aperture (NA) at 0.6, and writing power from 7-14 mW.

The reading conditions were: CLV at 2.5 m/s, wavelength at 405 nm, NA at 0.6, and reading power from 0.5 to 1.5 mW.

The tests provided for results within the expectations with regard to absorptivity, reflectance and recording sensitivity. Further, the obtained optical layers were sufficiently stable to light and heat. The optical recording media were storage stable within specification.

### EXAMPLES 9-14

Optical recording media with an optical layer comprising the dyes obtained according to examples 2 to 7 were manufactured and tested according to the procedure given for example 8.

As the results of examples 8 to 14 clearly indicate, the comounds of examples 1 to 7 are suitable for the use in optical layers for optical data recording media with very good results.

## Claims

1. Use of a mono- or dicationic naphthalene-based imide dye compound of the formulae (I), (II), (III) or (IV) in an optical layer for optical data recording, wherein the dye compound is selected from the group consisting of compounds of the formulae (I), (II), (III) and (IV) 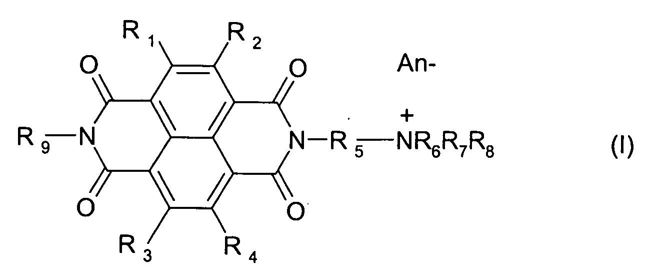 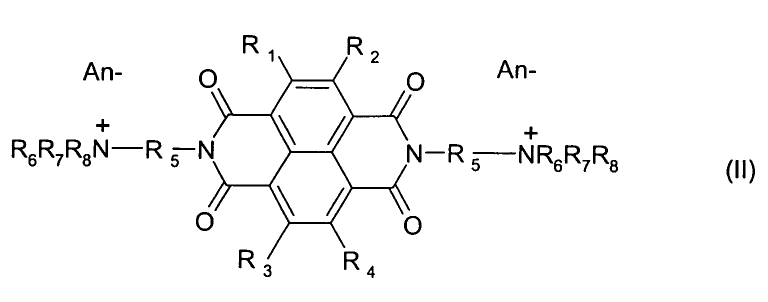 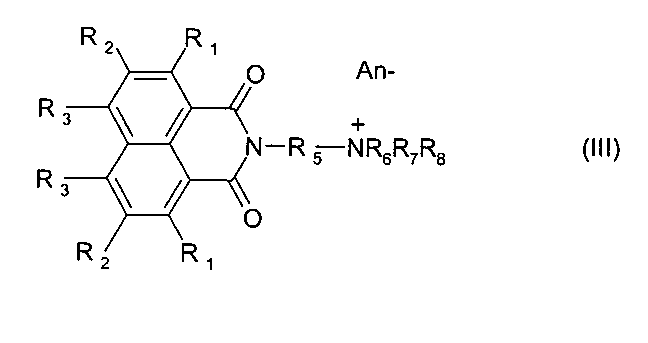 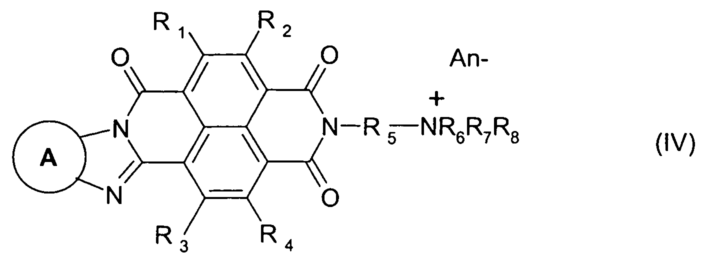 wherein
R₁, R₂, R₃, R₄ independently of one another, represent hydrogen, hydroxy (-OH), halogen (-F, -Cl, -Br, -I), cyano (-CN), nitro (-NO₂), C₁₋₈ alkyl, C₆₋₁₂ aryl, -NR₁₁R₁₂, C₁₋₈ alkyl, C₆₋₁₂ aryl, C₁₋₈ alkoxy, C₁₋₈ alkylthio, C₆₋₁₂ aryloxy, C₆₋₁₂ arylthio, -SO₃H, -SO₂R₁₁, - SO₂NR₁₁R₁₂, -CO₂R₁₁, -CONR₁₁R₁₂, -NHCOR₁₁, in which R₁₁ and R₁₂ are independently hydrogen or C₁₋₈ alkyl;
R₅ is C₁₋₂₀ alkylene, wherein the alkylene can be unsubstituted or substituted by C₁₋₈ alkoxy, C₁₋₈ alkylthio, C₆₋₁₂ aryloxy, C₆₋₁₂ arylthio, C₆₋₁₂ aryl;
R₆ is C₁₋₂₀ alkyl, wherein the alkyl can be unsubstituted or substituted by C₁₋₈ alkoxy, C₁₋₈ alkylthio, C₆₋₁₂ aryloxy, C₆₋₁₂ arylthio, C₆₋₁₂ aryl;
R₇, R₈ independently of one another, represent C₁₋₂₀ alkyl, wherein the alkyl can be unsubstituted or substituted by C₁₋₈ alkyl, C₁₋₈ alkoxy, C₁₋₈ alkylthio, C₆₋₁₂ aryloxy, C₆₋₁₂ arylthio, C₆₋₁₂ aryl; or are combined together to form C₃₋₆ cycloalkyl which can be unsubstituted or substituted by C₁₋₈ alkoxy, C₁₋₈ alkylthio, C₆₋₁₂ aryloxy, C₆₋₁₂ arylthio, C₆₋₁₂ aryl;
R₉ is is C₁₋₂₀ alkyl wherein the alkyl can be unsubstituted or substituted by C₁₋₈ alkyl, C₁₋₈ alkoxy, C₁₋₈ alkylthio, C₆₋₁₂ aryloxy, C₆₋₁₂ arylthio, C₆₋₁₂ aryl; or R₉ is C₆-aryl wherein the aryl can be unsubstituted or substituted by C₁₋₈ alkyl, C₁₋₈ alkoxy, C₁₋₈ alkylthio, C₆₋₁₂ aryloxy, C₆₋₁₂ arylthio; or R₉ represents a substituted or unsubstituted annealed aromatic ring system comprising at least one heteroatom in either the first or the second ring or in both, selected from one of the moieties (1) to (4): 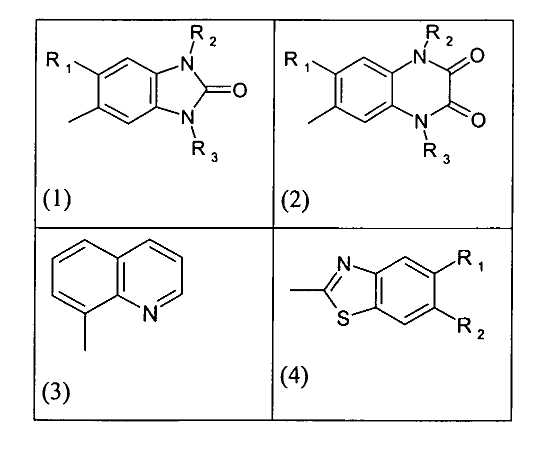
Nucleus A represents a substituted or unsubstituted annealed aromatic ring system comprising at least one heteroatom in either the first or the second ring or in both. A is selected from one of the moieties (5) to (8): 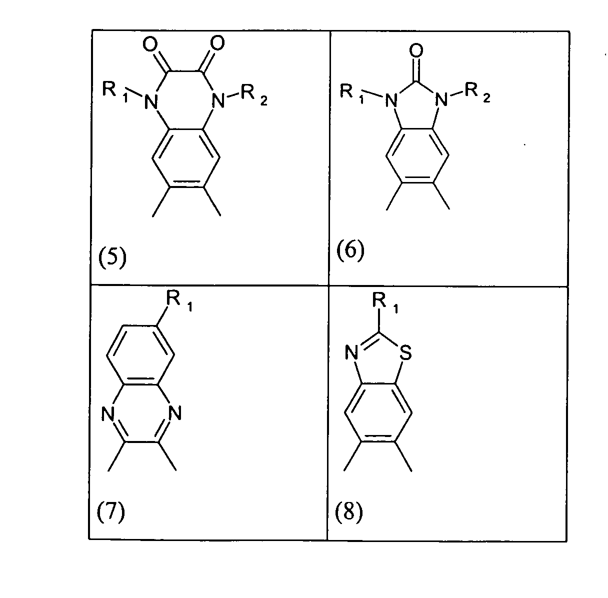
An⁻ represents an anion selected from halide, C₁₋₂₀ carboxylate, formiate, acetate, oxalate, lactate, glycolate, citrate, H₃C-SO₄⁻, ClO₄⁻, tetracyanoquinodimethane (TCNQ⁻), H₃C-SO₃⁻, ClO₄⁻, BX₄⁻, SbX₆⁻, PX₆⁻, in which X represents halogen (-F, -Cl, -Br, - I) or phenyl.

2. Use according to claim 1,wherein
R₁, R₂, R₃, R₄ independently of one another, represent hydrogen, halogen (-F, - Cl, -Br, -I), cyano (-CN), nitro (-NO₂), C₁₋₈ alkyl, C₆₋₁₂ aryl, hydroxy, C₁₋₈ alkoxy, C₆₋₁₂ aryloxy,
R₅ is C₁₋₆ alkylene, wherein the alkylene can be unsubstituted or substituted by C₁₋₆ alkoxy;
R₆ is C₁₋₆ alkyl, wherein the alkyl can be unsubstituted or substituted by C₁₋₈ alkoxy;
R₇, R₈ independently of one another, represent C₁₋₈ alkyl, wherein the alkyl can be unsubstituted or substituted by C₁₋₈ alkyl, C₁₋₈ alkoxy; or are combined together to form C₃₋₆ cycloalkyl which can be unsubstituted or substituted by C₁₋₈ alkyl or C₁₋₈ alkoxy;
R₉ is C₁₋₂₀ alkyl, wherein the alkyl can be unsubstituted or substituted by C₁₋₈ alkyl, C₁₋₈ alkoxy; or R₉ is a C₆ aryl unsubstituted or substituted by C₁₋₈ alkyl, C₁₋₈ alkoxy; or R₉ is a group selected from one of the moieties (1) to (4);
Nucleus A represents a substituted or unsubstituted annealed aromatic ring system comprising at least one heteroatom in either the first or the second ring or in both. A is selected from one of the moieties (5) to (8) wherein R₁, R₂, R₃ are defined as C₁₋₈ alkyl, C₁₋₈ alkoxy;
An⁻ represents an anion selected from tetracyanoquinodimethane (TCNQ⁻), BX₄⁻, SbX₆⁻, PX₆⁻, in which X represents halogen (-F, - Cl, -Br, -I) or phenyl.

3. An optical layer according to claim 1 comprising a mixture of dye compounds according to formulae (I), (II), (III) or (IV) as defined in claims 1 or 2.

4. A method for producing optical layers for a use according to claims 1 or 3, comprising the following steps
(a) providing a substrate
(b) dissolving a dye compound or a mixture of dye compounds of formula (I), (II), (III) or (IV) as defined in claims 1 or 3 in an organic solvent to form a solution,
(c) coating the solution (b) on the substrate (a);
(d) evaporating the solvent to form a dye layer.

5. A method according to claim 4, wherein the substrate is polycarbonate (PC) or polymethylmethacrylate (PMMA).

6. A method according to claim 4, wherein the organic solvent is selected from C₁₋₈ alcohol , halogen substituted C₁₋₈ alcohols, C₁₋₈ ketone, C₁₋₈ ether, halogen substituted C₁₋₄ alkane, or amides.

7. A method according to claim 4, wherein
the C₁₋₈ alcohols or halogen substituted C₁₋₈ alcohols are selected from methanol, ethanol, isopropanol, diacetone alcohol (DAA), 2,2,3,3-tetrafluoropropanol, trichloroethanol, 2-chloroethanol, octafluoropentanol or hexafluorobutanol;
the C₁₋₈ ketones are selected from acetone, methylisobutylketone, methylethylketone, or 3-hydroxy-3-methyl-2-butanone;
the halogen substituted C₁₋₄ alkanes are selected from chloroform, dichloromethane or 1-chlorobutane; and the amides are selected from dimethylformamide or dimethylacetamide.

8. A method according to claim 4, wherein the optical layer (dye layer) has a thickness from 70 to 250 nm.

9. An optical recording medium comprising an optical layer, wherein at least one dye compound of the formulae (I), (II), (III) or (IV) as defined in claim 1 is used.

10. A method for producing an optical recording medium according to claims 9, comprising the following steps
(a) providing a first substrate
(b) dissolving a dye compound or a mixture of dye compounds of formula (I), (II), (III) or (IV) as defined in claims 1 or 3 in an organic solvent to form a solution,
(c) coating the solution (b) on the first substrate (a),
(d) evaporating the solvent to form a dye layer,
(e) disposing a reflection layer on the dye layer and
(f) disposing a second substrate on the reflection layer.
